# EUROPEAN PATENT SPECIFICATION

(11) **EP 0 760 240 B1**
(45) Date of publication and mention of the grant of the patent: **12.10.2005**
(21) Application number: 96909178.4
(22) Date of filing: 10.04.1996
(51) Int. Cl.: A61K 31/52, A61K 35/78, A61K 31/4375, A61P 25/26

(54) **Composition for stimulating the cerebral activity comprising vinpocetine citrate and a plant extract comprising xanthines**
Zusammensetzung enthaltend Vinpocetin-Citrat und einen Xanthin-enthaltenden Pflanzenextrakt zur Stimulierung der Gerhirnaktivität
Compositions stimulatrice de l'activité cerebrale comprenant le citrate de vinpocetine et un extrait de plantes comprenant des xanthines

(30) Priority: 12.04.1995 ES 9500739
(43) Date of publication of application: 05.03.1997
(73) Proprietor: Decox, S.L., 28765 Tres Cantos (ES)
(72) Inventor: LAZARO FLORES, Consuelo, E-28765 Tres Cantos (ES); CALVO LAZARO, Paula, E-28765 Tres Cantos (ES); CALVO LAZARO, Elena, E-28765 Tres Cantos (ES); MANRESA FERRERO, Maria Teresa, E-28765 Tres Cantos (ES); CALVO MONDELO, Fernando, E-28765 Tres Cantos (ES)
(74) Representative: Pons Arino, Angel
(86) International application number: PCT/ES1996/000082
(87) International publication number: WO 1996/032113

(56) References cited:
- EP-A- 0 154 756
- FR-A- 2 193 586
- FR-A- 2 193 587
- FR-A- 2 284 325
- FR-A- 2 319 362
- US-A- 4 362 730
- EBERHARD TEUSCHER: "Pharmazeutische Biologie" 1983, PROF. DR. H.BORRISS/FRIEDR. VIEWEG *a SOHN, BRAUNSCHWEIG / WIESBADEN

## Description

### TITLE OF THE INVENTION

A new composition which stimulates cerebral activity, based upon alkaloids with an eburnamenine nucleus.

### TECHNICAL FIELD OF THE INVENTION

This invention relates to a new composition which stimulates cerebral activity, based upon the potent action which exhibits vinpocetine citrate when administered together with compounds with a xanthine structure.

### STATE OF THE ART

In general, alkaloids with an eburnane nucleus are sufficiently well-known peripheral vasodilators (US Patent 4,400,514, Spanish Patent 549.105), which act as cerebral oxygenators [Drug. Res., 26, 10a, 1947-1989 (1976)] and as memory activators [Drug. Res., 26, 10a, 1947-1950 (1976); Eur. J. Clin. Pharmacol., 28 567-571 (1985); Drug. Rev. Res., 14, 191-193 (1988); Inter. Clin. Psychopharmacology, 2, 325-331 (1987)]. Additionally, eburnamonine addition salts are known which exhibit properties analogous to the same, or greatly improved, and which, additionally, are soluble in aqueous solution, starting upon which it is possible to prepare drinkable solutions. As an example one may mention, for instance, vinpocetine citrate [European Patent 0154756], a cerebral oxygenator and vasodilator which exhibits greater bioavailability and less toxicity than vinpocetine, its starting base.

In relation to xanthine bases, we could mention, among the most widely known, theobromine which acts in others as a cardiotonic [A. Lelo et. al., Brit. J. Clin. Pharmacol., 22, 177 (1986)]. Another interesting xanthine base is propentofylline, which acts as a peripheral and cerebral vasodilator [K. Nagata et. al., Artznelmittel-Forsch, 35, 1034 (1985)], and as an inhibitor of adenosine [B.B. Fredholm et. al., Acta Pharmacol. Toxicol., 58, 187, (1986)], and as cognitive activator [I. Hindmarch, Z. Subhan, Ibid, 5, 379 (1985)], among others. Theophylline is a cardiac stimulant and a muscle relaxant [J.L. Cohen, Analytical Profiles of Drug Substances, Vol. 4, K. Florey, Ed. Academic Press New York, p. 466-493 (1975)]. Finally, caffeine is a sufficiently well known general stimulant which is extracted from tea and coffee.

### DESCRIPTION OF THE INVENTION

It has been known for some time that extracts or concoctions of some plants such as coffee, tea, cocoa, and ginseng, both that originating from Korea, as that from Manchuria in its different species, white or red, such as the one known as Siberian ginseng (Eleoterococus senticosus), or even oral administration of the same plant as it is found in nature, or ground with some further elaboration, have a characteristic tonic and stimulating effect which translates into a general invigoration of the physical state, together with a parallel stimulatory action over mental activity, which, depending on the plant in question, is more or less accentuated in relation to some of the sensations of physical well-being and/or mental alertness which are produced, with a greater and more relevant action depending upon the plant species.

The duration and the intensity of the stimulatory effect on the central nervous system (CNS) can be achieved either on the short term or on a longer term, depending on the concentration of the extract or on the plant species employed and, eventually, depending upon the amount and collection of active principles which are found in the plant.

The popular, and in some cases medical, use of these compounds in different forms has been known for many years. The sometimes undesirable side-effects obtained such as insomnia produced in some individuals due to coffee, or a certain degree of nervousness in some people which are very sensitive to chocolate, are also well known.

In any case, these effects are taken into consideration, which does not prevent the daily use of these products, which have an undoubted popular support in different areas all over the world.

In some countries the use of coffee is more extended, in all its varieties, such as arabic or robust, in others it is tea, ranging from green tea in Japan and China to the innumerable varieties of tea from India, Europe or the Arab nations.

Chocolate and the derivatives of the cocoa plant are widely implanted in the western world as stimulants of physical and psychic vigour.

There is extensive scientific literature, both chemical, pharmacological and medical, as well as traditional, which can illustrate what has been previously exposed, but we avoid providing references of the same due to the tremendous extension which such bibliography would require.

We believe that there is a great need for a composition which could be administered in the form of a drink, which had a stimulatory effect upon the central nervous system, in particular stimulating memory and cognitive activity, but which had fewer undesired effects than those observed with the usual drinks which are consumed on a daily basis. That is, being able to promote the stimulatory effect but reducing the undesired effect. The subject of the present invention is therefore a composition according to claims 1 and 2.

The active ingredients responsible for the stimulatory effects have been, in general, extensively identified, such that it is known that caffeine is one of the major activators of the mental alertness and well-being produced by coffee or tea, and that theobromine is responsible for the effect of cocoa and the derivatives of chocolate.

The actions encountered have been attributed to these xanthine bases, and even theophylline, which is closely related to caffeine and theobromine, is a member of the basic xanthine structure, which has very related properties.

Additionally, it has been observed that synthetically obtained compounds exhibit a great part of the properties of the natural extracts, being in some cases the dosification of their administration simpler.

The ginsenosides responsible for the invigoration produced by the administration of ginseng in its different forms and presentations have also been identified.

It can even be said that there is an important scientific armory which explains and relates the active principles produced by synthesis, or that are found in nature, as well as their pharmacologic activity.

On the other hand, the properties of the alkaloids with an eburnamonine structure are known due to their interesting effects upon cerebral circulation, upon cerebral vascular resistance in the blood vessels of the brain and their activity increasing cerebral metabolism, which can be quantified by the increase in glucose consumption by nerve cells, as well as by their greater resistance to cerebral hypoxia. From among the therapeutically employed alkaloids which correspond to this group, we could mention vinpocetine, vincamine, bromovincamine, eburnamonine or vincamone, among others, which are used with great success in the treatment of the deterioration of the cognitive functions in cerebrovascular disorders, which generally occur in people of an advanced age.

The authors have recently found an unexpected result after the oral administration, to healthy volunteers of different ages, of these traditional drinks in combination with the compound with an eburnamenine nucleus vinpocetine citrate. This effect has been achieved and optimized using different relative concentrations of the components, that is, making a gradation of the concentrations of the xanthine bases employed, and of the eburnamenine nucleus derivative in the case of coffee, tea and cocoa, or rather dosifying the concentration of the natural ginsenosides or the concentration of the active principles of *eleteorococus senticosus* in the case of the different ginseng extracts employed.

This marked and unexpected powerful synergic effect is obtained in all cases with the different concentrations or relative contents employed in each case, and in this way, it is possible to observe a possible adaptation of the optimum concentration to the individual need, measured as response in mental alertness, or in the increase in cognitive capacity, or also, as a measure of the reduction or absence of undesired effects.

Also, by means of the present invention it is possible to obtain compositions which give rise to effects on a shorter or longer term, thus being able to use these compositions in the form of drinks in a rational manner, stimulating activity and vigour, both physical and mental, on the short, medium or long term, choosing, in accordance to the needs and individual sensitivity to the composition, the optimum effect.

In order to try to explain these effects which seem to take place on the short term and that also have durations in the medium and long term, a number of hypotheses have been pointed out, centered, generally, upon the basic knowledge which exists in relation to the biochemistry of nerve cells.

We will expose, in a succinct manner, some of the facts and their possible explanation, in relation to the synergic phenomenon which takes place.

Thus, for instance, it is known that vinpocetine reduces or inhibits phosphodiesterase PDE, in the same way as theophylline does so to a lesser extent. Other xanthines can also do this, such as caffeine or theobromine.

Adenyl cyclase acts upon the acid adenosine monophosphate (AMP) yielding cyclic adenosine monophosphate (cAMP), which by the action of PDE, liberates AMP again (see French Patent 80 17165, Publication No. 2469180). Therefore, if theophylline inhibits the action of phosphodiesterase (PDE), and hence inhibits the formation of cAMP, and vinpocetine also does this, there is an interesting synergic effect to consider and use in the global activation of cerebral activity. This can be measured very effectively by the methods described in the literature (Biochem. Biophys. Acta 302, 50/1973, etc.) on animal tissue homogenates, for example rat's brains, being it possible to perfectly tabulate and measure the inhibitory effect of several concentrations of the purified enzyme (PDE) versus cAMP of the substrate, which can be determined quantitatively. If vinpocetine is added to the different solutions of varying concentration of PDE it is possible to observe how it acts, surprisingly revealing a clear inhibition of the enzymatic activity of PDE over the reference substrate. In conclusion, this synergy can be used as, due to it, AMP is increased at the expense of cAMP, therefore there being a greater input of energy originating from the ATP which is formed as a consequence of the greater availability of non-cyclic AMP, which in turn allows a remarkable increase in intracellular metabolic processes, in particular in the nerve cell, with the corresponding increase in glucose consumption and the start-up of the biochemical processes of fat mobilization and use of the available glucose, due to the greater availability of the necessary energy, to accelerate, activate or implement these processes where, precisely in the brain, energy resources are very limited due to the fact that the energy source originating from mobilizable polysaccharides, which in every case are controlled by a very precise mechanism for the obtention of the same, is also very limited.

The ATP produced in this manner is the source of energy available for the biochemistry of the brain.

In other eburnanes, vinpocetine was shown to also increase the levels of noradrenaline and dopamine in the brain.

We precisely activate those transmitters which are specifically in charge of the biochemistry of the brain, and associating this to the greater obtention of cellular oxygen provided by the proven anti-hypoxic action of vinpocetine, it is possible to obtain data which is measurable by experimentation, such as the measurement of the increase in the cerebral concentration of liberated serotonin (5HT) as well as a clear increase of its metabolite 5-hydroxyindole acetic acid (5HIAA). This effect is at its maximum between 2 and 4 hours after the administration.

The described synergic use of plant extracts comprising xanthine bases and vinpocetine citrate, administered orally, produce, in summary, an increase in the concentration of neurotransmitters and an increase in ATP, which gives rise to an effective alteration of the cerebral metabolism which is subjectively and objectively quantifiable.

Let us remember that it has been proven that compounds such as vinpocetine, theophylline and papaverine inhibit PDE whereas norepinephrine stimulates adenyl cyclase, with its subsequent effect upon the concentrations of AMP.

It can be said that we obtain parallel effects by inhibiting PDE or stimulating the function of adenyl cyclase (ADC), in what regards to the mobilization or immobilization of AMP according to the scheme,
AMP → cAMP → AMP, and in parallel, AMP → ATP → ENERGY + AMP → ATP etc.

These phenomena permit supposing that they increase the consumption of glucose in the brain and that they activate cerebral metabolism, as it is possible to determine thanks to the modern brain scanning techniques when they are applied on people who are performing certain cerebral functions after the consumption of the drinks which are presented here, over adequate periods, versus subjects which have not been treated.

### EMBODIMENTS OF THE INVENTION

A better understanding of the process of the invention will be attained by means of the examples for its obtention that follow.

### EXAMPLE 1

Within a mixer-homogenizer suited to the effect, 500 kg of dry extract of granulated or non-granulated coffee and 250 g of vinpocetine citrate are conveniently mixed, obtaining a good degree of homogenization. This mixture is subsequently packaged in airtight sachets or airtight plastic and/or single dose aluminium containers, in the absence of humidity, containing the appropriate amount for a single dose, thus obtaining containers with a net weight of approximately 5 g each, to which approximately 150 to 200 mL of water can be added to obtain a drink which tastes like coffee and which is susceptible of admitting sugar, edulcorants or additives to taste.

### EXAMPLE 2

Within a mixer suited to the effect, 100 kg of tea extract, of any of its varieties ranging from green tea of the kind generally used in Japan, or Chinese Oolong tea or Indian black tea or any tea for infusion which is usually on the market, and 100 g of vinpocetine citrate are conveniently mixed, subsequently proceeding to its packaging and use as described in the previous example, preferably employing water in the final drink.

### EXAMPLE 3

In a manner analogous to that described in example 1, but using an extract of decaffeinated coffee.

### EXAMPLE 14

Within a stirred vessel, 200 L of diluted cola extract, based upon cola extracts of the kind commonly found in the soft-drinks market, are introduced together with 2.5 liters of concentrated and purified extract of *Vinca minor* or with dehydrogenated extract of *Crioceras longiflorus* (apocinaceae). This is stirred and water is subsequently added until the concentration of alkaloids is adjusted to 15 mg per 100 mL. It is convenient to carry out this determination by the techniques commonly employed in the analytical determination of these alkaloids (i.e. quantitative HPLC). The solution obtained in this manner can be carbonated with carbon anhydride and bottled, in a manner analogous to that commonly used in the soft-drinks industry, in containers made of glass or of aluminium interiorly coated with a plastic film, or in a tetrabrik type container, that is cardboard interiorly coated with a plastic film, or in suitably treated tinplate containers as those used in the fruit juice industry. The expiry date must be stated in the container after performing the appropriate stability at room temperature tests and accelerated ageing tests, as is mandatory in the industries to this effect.

A stability study on the resulting drinks has been carried out in parallel, proving that both the organoleptic properties, as well as the product, the vinpocetine addition salt, or the eburnane extract, remain stable within the limits of stability defined for each type of drink.

## Claims

1. A composition for oral use which stimulates the activity of the brain, **characterised in that** it comprises a mixture formed by:
a) vinpocetine citrate; and
b) a drink based on extracts of coffee, tea, cocoa, mate grass or cola;
said mixture comprising between 0,5 and 30 mg of vinpocetine citrate per each 100 ml of said extract-based drink.

2. A composition for oral use which stimulates the activity of the brain, **characterised in that** it comprises a mixture formed by:
a) vinpocetine citrate; and
b) a plant extract for extract-based drinks selected from extracts of coffee, tea, cocoa, mate grass and cola, said extract being dry, semi-dry or wet, or granulated, powdered, fluid, pasty or semi-pasty;
said mixture comprising between 0,5 and 30 mg of vinpocetine citrate per each 5,20 gr of said extract for extract-based drinks.

## Patentansprüche

1. Zusammensetzung zum Einnehmen zur Anregung der Gehimtätigkeit, bestehend aus einer Mischung aus
a) Vinpocetincitrat, sowie
b) einem Getränk auf der Basis eines Extraktes aus Kaffee, Tee, Kakao, Mateteestrauch oder Kola, mit einem Vinpocetincitratgehalt von 0,5 - 30 mg pro 100 ml des besagten Extraktgetränks.

2. Zusammensetzung zum Einnehmen zur Anregung der Gehimtätigkeit, bestehend aus einer Mischung aus
a) Vinpocetincitrat, sowie
b) einem Pflanzenextrakt für Getränke auf der Basis ausgewählter Extrakte aus Kaffee, Tee, Kakao, Mateteestrauch und Kola, in trockener, halbtrockener oder hydrierter Form bzw. als Granulat, Pulver, Flüssigkeit oder dicke oder halbflüssige Paste; mit einem Vinpocetincitratgehalt von 0,5 - 30 mg pro 5,20 g Extrakt.

## Revendications

1. Composition pour usage oral stimulant l'activité cérébrale, **caractérisée par le fait qu'**elle comprend un mélange formé par
a) du citrate de vinpocétine et
b) une boisson à base d'extraits de café, de thé, de cacao, de maté ou de cola comprenant entre 0,5 et 30 mg de citrate de vinpocétine pour 100 ml de la boisson en question à base d'extraits.

2. Composition pour usage oral stimulant l'activité cérébrale, **caractérisée par le fait qu'**elle comprend un mélange formé par
a) du citrate de vinpocétine et
b) un extrait de plante pour boissons à base d'extraits sélectionnés à partir d'extraits de café, de thé, de cacao, de maté et de cola, ledit extrait étant sec, demi-sec, hydraté, granulé, en poudre, fluide, en pâte épaisse ou demi-liquide ; ledit mélange comprend entre 0,5 et 30 mg de citrate de vinpocétine pour 5,20 g dudit extrait pour boissons à base d'extraits.
